# EUROPÄISCHE PATENTSCHRIFT

(11) **EP 1 147 745 B1**
(45) Veröffentlichungstag und Bekanntmachung des Hinweises auf die Patenterteilung: **09.06.2004**
(21) Anmeldenummer: 01109650.0
(22) Anmeldetag: 19.04.2001
(51) Int. Cl.: A61B 17/66, A61C 7/10

(54) **Vorrichtung für die Gaumenerweiterung**
Device for expansion of the palate
Dispositif pour l'expansion du palais

(30) Priorität: 20.04.2000 CH 8922000
(43) Veröffentlichungstag der Anmeldung: 24.10.2001
(73) Patentinhaber: Triaca, Albino, 8006 Zürich (CH); Merz, Beat, 4133 Pratteln (CH); Minoretti, Roger, 8006 Zürich (CH)
(72) Erfinder: Triaca, Albino, 8006 Zürich (CH); Merz, Beat, 4133 Pratteln (CH); Minoretti, Roger, 8006 Zürich (CH); Egli, Thomas, 8123 Ebmatingen (CH)
(74) Vertreter: Grimm, Ekkehard, Dipl.-Phys.

(56) Entgegenhaltungen:
- DE-A- 19 921 822
- US-A- 597 582
- US-A- 5 620 321

## Beschreibung

Die vorliegende Erfindung betrifft eine Vorrichtung für die Gaumenerweiterung mit einem in seiner Längenabmessung verlängerbaren und/oder verkürzbaren Extensionsteil, das an seinen beiden Enden jeweils ein Fixierteil aufweist.

Eine solche Vorrichtung, auch als Distraktor bezeichnet, ist eine kieferorthopädischkieferchirurgische Apparatur, die es erlaubt, durch graduelle, d.h. schrittweise Extension den Oberkiefer in der Breite rasch auszudehnen oder zwei andere Knochensegmente graduell, im Sinne einer Distraktion, auseinander zu bewegen. Die Apparatur kann auch bei einer Verbreiterung des Oberkiefers in einem Schritt benutzt werden, um die Position der beiden Gaumenhälften zueinander und zum Unterkiefer präzise einstellen zu können. Damit können Okklusionsstörungen, Malformationen, Knochenasymmetrien und Missverhältnisse zwischen Ober- und Unterkiefer korrigiert werden, welche angeboren oder erworben sein können.

Bei der ersten Hauptanwendung, der sogenannten raschen Gaumenerweiterung, geht es darum, eine ungenügende Breite des Oberkiefers durch eine kieferorthopädischkieferchirurgische Behandlung so zu korrigieren, dass das Verhältnis zwischen Oberkieferund Unterkieferzahnbogen normalisiert wird. Der Oberkiefer besteht dabei aus einer linken und einer rechten Hälfte, die in der mittsagittalen Ebene in der Gaumennaht vereint sind. Bei Patienten unter dem Alter von 12-14 Jahren ist diese Verbindung im allgemeinen noch nicht grossflächig verknöchert, so dass durch graduelles Auseinandertreiben der zwei Hälften die orthognathische Korrektur einfach durchführbar ist. Bei älteren Jugendlichen oder Erwachsenen ist die Naht in einem zunehmenden Masse bis total zusammengewachsen. In diesen Fällen ist oft eine chirurgische Unterstützung notwendig, indem Teile des Oberkiefers so geschwächt werden, dass die Naht erneut aufgesprengt werden kann, um anschliessend die graduelle Verbreiterung durchzuführen.

In der zweiten Hauptanwendung, der konventionellen Distraktion von Knochensegmenten in einer beliebigen Richtung, geht es darum, Knochenabmessungen, die zu gering sind, zu vergrössern. Ein typisches Beispiel ist der zu kurze horizontale Teil des Unterkiefers, der in einem Overjet (Überbiss) resultiert, mit den dazugehörigen kieferorthopädischen Komplikationen und den ästhetischen Problemen bezüglich des Gesichtsprofils. In einem kieferchirurgischen Eingriff wird der Knochen durchtrennt und durch den Distraktor überbrückt. Nach einer kurzen Anheilungsphase von ein paar Tagen wird das die Trennstelle überbrückende Heilungsgewebe, der Kallus, graduell distrahiert, d.h. die Knochenkompartimente mittels des Distraktors auseinanderbewegt.

In einer weiteren Anwendung werden solche Geräte nur intraoperativ benutzt, um die Position von Teilen des Oberkiefers gegeneinander präzise einstellen zu können, z.B. im Fall einer Lippen-Kiefer-Gaumenspalte. Diese Einstellung ist ohne eine solche Hilfe sehr schwierig, wegen der durch die Weichteile ausgeübten Zugkräfte. Bei Erreichen der gewünschten Position nach Kontrolle der Okklusion mit dem Unterkiefer werden die Knochenteile in der erzielten Position festgeschraubt und das Gerät wird anschliessend wieder entfernt.

Die Hauptgruppe der heute klinisch angewandten Geräte ist jene der auf den Zähnen abgestützten Apparaturen. Diese haben einige gewichtige Nachteile.

Eine weitere Möglichkeit ist die Abstützung auf Gaumenplatten. Hierbei ist ein einigermassen präzises Steuern der Bewegungen sehr schwierig, wegen der indirekten Kraftübertragung über die Schleimhäute. Unter den Druckstellen kann es zu Entzündungen und hygienischen Problemen kommen.

Bekannte Distraktoren der vorstehend angegebenen Art, die auf den Zähnen abgestützte Apparaturen sind, sind beispielsweise in der EP 0919207 A1, der WO 94/26196 und der US 3,977,082 beschrieben. Distraktoren für die Gaumenerweiterung, die sich über Gaumenplatten direkt auf der Gaumenschleimhaut abstützen, sind in der EP 0308645 A1 beschrieben.

Schliesslich beschreibt die WO 94/10933 einen Distraktor, der sich sowohl auf Zähnen als auch auf Gaumenplatten abstützt.

DE 19921822 offenbart einer Distraktor gemäß dem Oberbegriff des Anspruchs 1.

Ein Nachteil der parodontalen Abstützung besteht auch darin, dass die zwei Gaumenhälften bei den bestehenden Abstützungen in der frontalen Ebene kippen können anstatt sich in einer stabilen Winkellage in der Frontalansicht auseinanderzubewegen.

Ein weiterer Nachteil des dargestellten Standes der Technik ist darin zu sehen, dass die bestehenden Apparaturen lediglich eine einachsige, parallele Verschiebung der zwei Gaumenhälften ermöglichen. In der praktischen Anwendung ist es aber oft nötig, eine nicht parallele Verschiebung durchzuführen, z.B. indem der hintere Gaumen mehr aufgeweitet werden muss als der vordere Gaumen. Ebenfalls wichtig ist, dass das Bedürfnis nach unterschiedlichen Vektoren im hinteren und vorderen Gaumen oft erst im Laufe der Behandlung erkennbar wird, weil z.B. die Widerstände hinten und vorne ungleich sind.

Die Abstützung auf den Zähnen belastet den Zahnhalteapparat, das Paradont. Dies kann unter Umständen zu Wurzelresorptionen führen. Des weiteren ist eine ungewollte Kippung der Zähne nicht auszuschliessen, so dass schliesslich nicht der knöcherne Gaumen erweitert wird, sondern lediglich die Zähne nach lateral abgekippt werden. Tatsächlich gibt es in der Literatur Hinweise, dass bis zu 75% der vermeintlichen Bewegung effektiv dentaler Natur ist, d.h. dass die Zähne verkippt sind oder sich nach lateral bewegt haben, was wiederum zur Gefahr der Freilegung der Wurzeln oder zur Bissöffnung führt. Bei erwachsenen Patienten mit parodontal geschädigten oder ausgefallenen Zähnen oder bei jugendlichen Patienten mit Nichtanlagen von Zähnen ist eine Abstützung auf den Zähnen a priori mehr als problematisch, da der übriggebliebene Zahnhalteapparat die Kräfte nicht unbeschädigt übertragen kann.

Der vorliegenden Erfindung liegt die Aufgabe zugrunde, einen Distraktor zu schaffen, der die vorstehend angeführten Nachteile bekannter Distraktoren, insbesondere solcher, die sich auf den Zahn abstützen, zumindest teilweise vermeidet.

Gelöst wird diese Aufgabe durch eine Vorrichtung für die Gaumenerweiterung mit einem in seiner Längenabmessung verlängerbaren und/oder verkürzbaren Extensionsteil, das an seinen beiden Enden jeweils ein Fixierteil aufweist, die dadurch gekennzeichnet ist, dass jedes Fixierteil als punktförmiges Knochenabstützungsteil aufgebaut ist.

Ein solcher Distraktor vermeidet Gaumenplatten oder Abstützungen auf den Zähnen, die die vorstehend angegebenen Nachteile aufweisen, indem sich das Expansionsteil über die Fixierteile knöchern und punktförmig abstützt. Hierbei werden durch eine Stichinzision in der Gaumenschleimhaut entweder spezielle Verankerungsschrauben in den Knochen geschraubt oder die Abstützungsteile sind als Dorn ausgebildet. Solche Abstützungsteile können knochenseitig praktisch an beliebigen Positionen angesetzt und damit fixiert werden, und zwar unabhängig der jeweiligen Position der Zähne, die in keinster Weise als Haltemittel für solche Extensionsteile benötigt werden. Auch müssen keine speziellen Anpassungen für den jeweiligen Patienten vorgenommen werden, wie dies beispielsweise bei Gaumenplatten oder Zähnen der Fall ist. Schliesslich ist es auch möglich, zwei der Distraktoren an definierten Stellen zu fixieren, um bestimmte Kraftkomponenten bei der Gaumenerweiterung auf den knöchernen Bereich des Gaumens ausüben zu können.

Es ist auch möglich, eine hintere und eine vordere, voneinander unabhängige Distraktionseinheit einzusetzen, so dass durch unterschiedliche Extension eine nicht parallele Verschiebung möglich wird. Hierbei können die Geräteachsen so gelegt werden, dass nicht nur eine Verbreiterung des Gaumens, sondern gleichzeitig eine Verlängerung erfolgt, falls dies nötig ist.

In einer Ausgestaltung kann das Knochenabstützungsteil mit dem Extensionsteil über ein einstellbares und in seiner Position fixierbares Gelenk verbunden sein; bei dieser Verbindung zwischen Extensionsteil und Knochenauflage/Schraube kann ein Moment übertragen werden, falls dies erforderlich ist, indem in der einen Variante das Gelenk auf der Befestigungseinheit festgeklemmt wird, während es in einer andern Variante starr ist.

Um zum einen die Eindringtiefe des Knochenabstützungsteils zu begrenzen und darüber hinaus die Druckbelastungen auf den Knochen flächenmässig zu verteilen, kann eine die Eindringtiefe des Abstützungsteils begrenzende Verdickung vorgesehen werden. Eine solche Verdickung ist sowohl bei geschraubten Knochenabstützungsteilen als auch bei als Dorn ausgebildeten Knochenabstützungsteilen von Vorteil.

Derartige Verdickungen, die in einer weiteren Ausgestaltung tellerartig oder wulstartig ausgebildet sind, haben den zusätzlichen Vorteil, dass sie in der Schleimhaut einwachsen können, so dass sich ein Formschluss ergibt. Ausserdem hat dieses Einwachsen den Vorteil, dass das Knochenabstützungsteil so in Position gehalten wird und nicht abfallen kann.

In einer konstruktiven Ausgestaltung des Extensionsteils sind dafür mindestens drei Gewindeelementen vorgesehen, die miteinander verschraubt zwei gegenläufige Gewindepaare bilden, wobei eine Drehung des mittleren Gewindeelements in der einen bzw. der anderen Drehrichtung das Extensionsteil verlängert oder verkürzt. Solche Extensionsteile können trotz ihres einfachen Aufbaus sehr einfach verlängert oder verkürzt werden; der Betrag einer Verlängerung oder einer Verkürzung pro Umdrehung der Gewinde kann darüber hinaus durch die Gewindesteigung vorgegeben werden.

In einer alternativen, konstruktiven Ausgestaltung ist das Extensionsteil aus mindestens drei Elementen aufgebaut. Diese drei Elemente sind miteinander verbunden, wobei das erste Element hülsenförmig ausgebildet und darin drehbar gelagert das zweite Element aufnimmt. Das dritte Element ist mit dem zweiten Element so verschraubt ist, dass unter Drehung des zweiten Elements in die eine bzw. in die andere Richtung das dritte Element in das erste Element hineingezogen oder aus diesem herausgeschoben wird und damit das Extensionsteil verkürzt oder verlängert wird.

Ein Aufbau des Distraktors so, dass die Achsen der Knochenabstützungsteile zu der Achse des Extensionsteil versetzt sind, bringt den Vorteil mit sich, dass auch die Knochenabstützungsteile durch entsprechende Halterungselemente vorab eingestellt oder nachgestellt werden können. Hierzu kann das jeweilige Klemmteil an dem Extensionsteil lösbar und verschiebbar angeordnet werden, so dass das jeweils darin gehaltene Knochenabstützungsteil zusammen mit diesem Klemmteil verschoben werden kann. In einer weiteren Ausführung der Vorrichtung wird auch das Klemmteil an dem Extensionsteil lösbar und verschiebbar angeordnet.

Gerade bei einer Anordnung mit zwischen den Knochenabstützungsteilen und dem Extensionsteil versetzten Achsen kann an demjenigen Ende bzw. der Stirnfläche des Knochenabstützungsteils, das dem sich an den Knochen anlagernden Ende gegenüberliegt, eine Druckfläche vorgesehen ist, an der ein entsprechendes Werkzeug angesetzt wird. Ein solches Werkzeug kann eine Zange sein, die zwischen die beiden sich gegenüberliegenden Druckflächen der beiden Knochenabstützungsteile zwischengefügt und gespreizt wird, so dass die Knochenabstützungsteile in Richtung des Knochens hin verschoben werden. Mit dieser Anordnung kann beispielsweise eine Grobeinstellung vorgenommen werden, um den Distraktor zu positionieren, während dann der Feintrieb bei der Extension über die Gewindestangen vorgenommen wird. Eine solche Druckfläche an dem Knochenabstützungsteil kann als Vertiefung ausgebildet werden, so dass ein Abrutschen eines entsprechenden daran anliegenden Werkzeugs verhindert wird.

Bevorzugt wird das Gelenk, mit dem das jeweilige Knochenabstützungsteil an dem Extensionsteil verbunden ist, durch ein Kugelgelenk gebildet werden, wobei die Kugel des Gelenks dem Knochenabstützungsteil zugeordnet ist, während das Extensionsteil eine entsprechende Kugelpfanne zur Aufnahme dieser Kugel und zum Klemmen und Halten der Kugel aufweist. Gerade ein solches Kugelgelenk, das entweder drehbar gehalten ist oder in der Lagerpfanne verklemmt werden kann, ermöglicht eine Einstellung des Distraktors so, dass sich die Knochenabstützungselemente nicht nur linear auseinanderbewegen. In einer Varianten kann der Kugelkopf zwischen der knöchernen Abstützung und dem Extensionsteil nicht angezogen werden bzw. temporär gelockert werden, wodurch sich dann die Knochensegmente in ihrem Winkel zueinander leicht anpassen lassen und damit zum Beispiel die Kiefergelenkköpfchen bei der Mittellinien-Distraktion des Unterkiefers in der Pfanne verbleiben, so dass damit keine Probleme in den Kiefergelenken des Patienten auftreten.

Die vorstehend erwähnten Klemmteile können so ausgelegt sein, dass sie jeweils ein Gewindeelement des Extensionsteils bilden. Hierzu kann das jeweilige Gewindeelement des Klemmteils eine Hülse mit Innengewinde sein, in die sich eine entsprechende Gewindestange einschraubt.

Weitere Einzelheiten der Erfindung ergeben sich aus der nachfolgenden Beschreibung von Ausführungsbeispielen anhand der Zeichnungen.
- Fig. 1a: zeigt eine Draufsicht auf den knöchernen Gaumen mit zwei Distraktoren zur schnellen Gaumennahterweiterung, wobei sich die Distraktoren mit speziellen Knochenschrauben abstützen; die Anordnung ist anhand eines Gipsmodells demonstriert.
- Fig. 1b: zeigt einen im Oberkiefer eingesetzten Distraktor entsprechend der Ausführungsform der Fig. 1a in einem Frontalschnitt (wiederum dargestellt an einem Gipsmodell).
- Fig. 2: zeigt einen asymmetrischen Oberkiefer, anhand eines Gipsmodells, der durch eine schräge Verlagerung eines Teils des Alveolarfortsatzes in die gewünschte Form gebracht wird; hierbei dient der Distraktor zur präzisen intraoperativen Einstellung der Knochensegmente.
- Fig. 3a: zeigt eine Distraktion eines Oberkiefersegments samt dazugehöriger Zähne gegen anterior, und zwar in einer Darstellung am Gipsmodell.
- Fig. 4: zeigt eine Distraktion eines Segments des posterioren, harten Gaumens gegen dorsal; wiederum dargestellt anhand eines Gipsmodells.
- Fig. 5: zeigt eine Distraktion des Oberkiefer-Alveolarfortsatzes gegen anterior, darstellt anhand eines Gipsmodells.
- Fig. 6a: zeigt eine Distraktion in einer Draufsicht auf den knöchernen Gaumen, wobei der Distraktor Knochenabstützungsteile in Form von speziellen Verankerungsbügeln aufweist; wiederum dargestellt anhand eines Gipsmodells.
- Fig. 6b: zeigt eine Frontalansicht des linken Verankerungsbügels der Fig. 6a.
- Fig. 7a: zeigt eine seitliche Ansicht eines weiteren Distraktors mit Klemmteilen;
- Fig. 7b: zeigt die Draufsicht auf das Klemmteil entsprechend des Sichtpfeils A in Fig. 7a.
- Fig. 8: zeigt eine vergrösserte Ansicht einer Kugelkopfschraube.
- Fig. 9: zeigt eine vergrösserte Ansicht eines Verankerungsbügels.
- Fig. 10: zeigt eine weitere Ausführungsform eines Distraktors mit speziellen Knochennägeln bzw. Knochenplatten zur Abstützung am Knochen.
- Fig. 11a und 11b: zeigen seitliche Ansichten eines weiteren Distraktors in einer zusammengeschobenen und einer ausgedehnten Position.
- Fig. 12a und 12b: zeigen eine weitere Variante eines Distraktors in den zwei unterschiedlichen Positionen, die den Positionen der Fig. 11a und 11b entsprechen.
- Fig. 12c: zeigt eine Draufsicht auf die Anordnung der Fig. 12b aus Richtung des Sichtpfeils C.
- Fig. 13: zeigt ein Klemmteil, wie es auch in den Fig. 12a und 12b zu sehen ist, wobei anstelle eines Abstützungsteils mit einer Kugel und einem Gewindeelement ein dornartiges Abstützungsteil gehalten ist, das darüber hinaus in dem Klemmteil verschiebbar ist.
- Fig. 14a, 14b und 14c: zeigen eine weitere Anordnung, wobei diese Figuren mit den Fig. 12a, 12b und 12c vergleichbar sind.
- Fig. 15: zeigt einen Dorn als Klemmteil, das anstelle der schraubbaren Klemmteile mit Kugelkopf in der Ausführungsform der Fig. 14a und 14b einsetzbar ist.

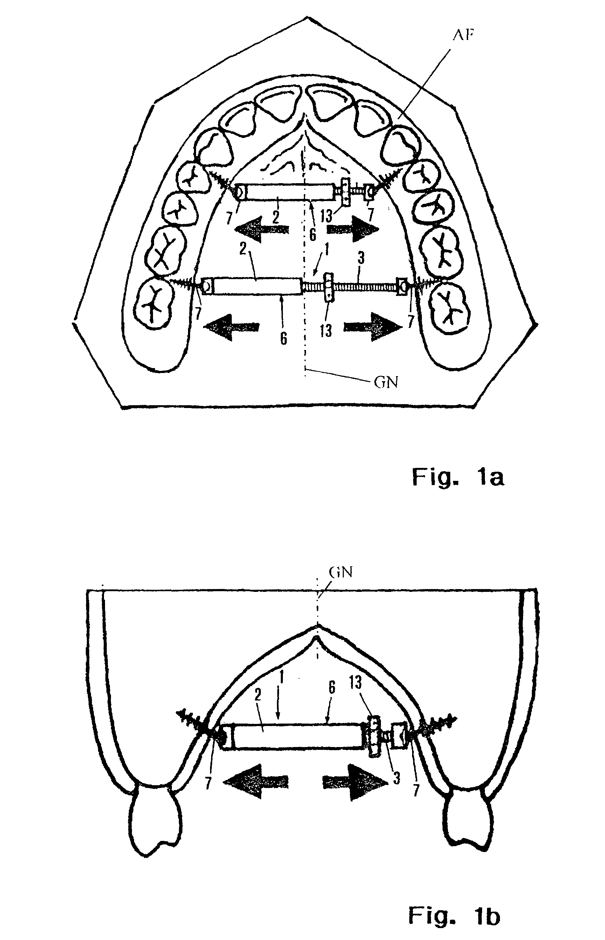

In Figur 1a ist in einer Draufsicht auf ein Gipsmodell die Verankerung von zwei Distraktoren gemäss einer ersten Ausführungsform gezeigt, wobei einer der Distraktoren auch in einer Ansicht von vorne in Figur 1b gezeigt ist.

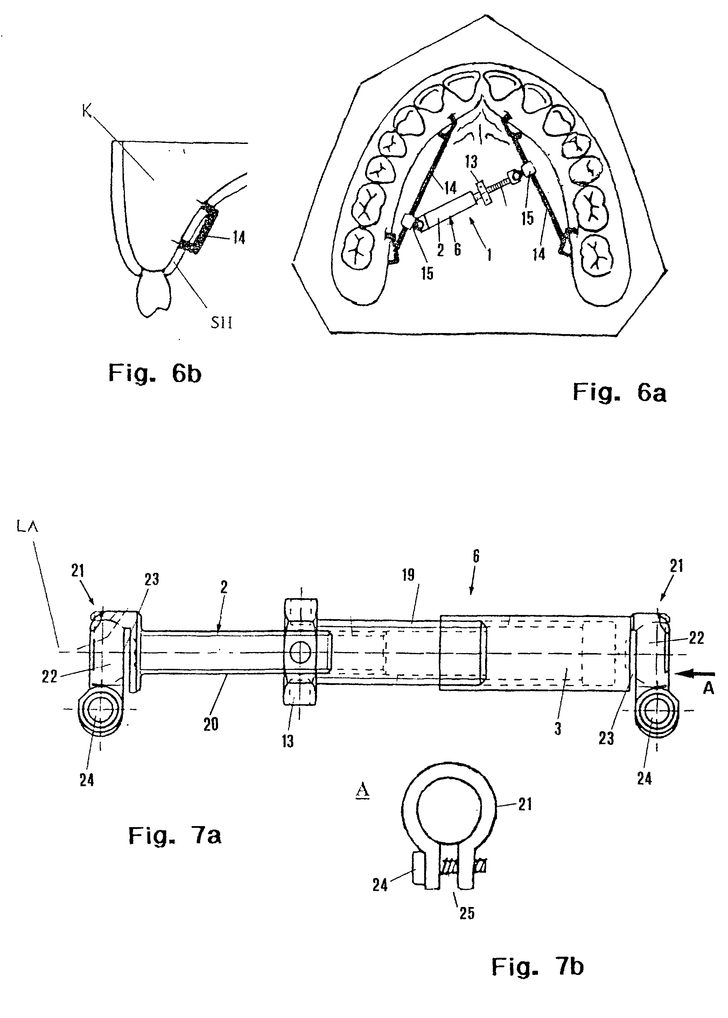

Dieser Distraktor 1, wie er in seinem Grundaufbau auch detaillierter in Figur 7a gezeigt ist, umfasst zwei Gewindeelemente 2, 3, wobei das eine Gewindeelement 3 ein hülsenförmiges Teil mit Innengewinde ist, während das andere Gewindeelement 2 in Form einer Gewindestange aufgebaut ist. An dem Ende der Gewindestange 2 ist Gelenkpfanne 4 befestigt; eine entsprechende Gewindepfanne 5 ist auch an dem Ende der Gewindehülse 3 angeordnet. Die beiden Gewindeelemente 2, 3 bilden ein in seinen Längenabmessungen verlängerbares und/oder verkürzbares Extensionsteil, das allgemein mit dem Bezugszeichen 6 bezeichnet ist. In den jeweiligen Gelenkpfannen 4, 5 an den Enden des Extensionsteils 6 ist ein Knochenabstützungsteil in Form einer Knochenschraube 7 eingesetzt; eine solche Knochenschraube 7 ist in einer vergrösserten Darstellung gezeigt. Diese Knochenschrauben 7 umfassen einen Gewindeteil 8, der mit einem Abstützungsring bzw. einer Abstützungsscheibe 9 abschliesst. An diese Abstützungsscheibe 9 schliesst dann ein Halbteil 10 an, an das sich dann eine Gelenkkugel 11 anschliesst. Im Kopfbereich der Gelenkkugel 11 ist ein Schlitz oder Kreuzschlitz 12 vorhanden (oder ein Innen-Mehrkant), um diese Knochenschraube 7 mit einem kieferchirurgischen Standardschraubendreher in den Knochen einzusetzen, wie dies in den Figuren 1a und 1b gezeigt ist. Die Abstützungsscheibe 9 begrenzt dabei die mögliche Eindringtiefe der Knochenschraube 7. Das Halbteil 10 dient zur Überbrückung der Schleimhaut, so dass die Kugel 11 ausserhalb der Schleimhaut gut zugänglich ist.

Eine erste Anwendung der Distraktoren 1 liegt in der schnellen, transversalen Aufdehnung der Gaumennaht, die mit GN in den Figuren 1a und 1b bezeichnet ist. Hierzu werden zwei Distraktoren, wie dies in Figur 1a gezeigt ist, jeweils im vorderen und im hinteren Bereich des Gaumens eingesetzt und auf den zwischen den Zahnwurzeln eingeschraubten Knochenschrauben 7 abgestützt. Die jeweiligen Extensionsteile 6 der beiden Distraktoren können dann verlängert werden, indem das Gewindeelement bzw. die Gewindestange 3 mittels einer daran drehfest angeordneten Sechskantmutter 13 gedreht wird und sich dadurch aus dem Innengewinde des hülsenförmigen Gewindeleements 2 herausdreht. Diese Distraktion mit zwei Geräten ermöglicht eine nicht parallele Aufdehung des Gaumens, um den oberen Alveolarfortsatz, mit AF bezeichnet, samt den darin befindlichen Zähnen genau auf die Unterkieferbezahnung abzustimmen. In einfacheren Fällen kann die Gaumenerweiterung mit nur einem Gerät erfolgen.

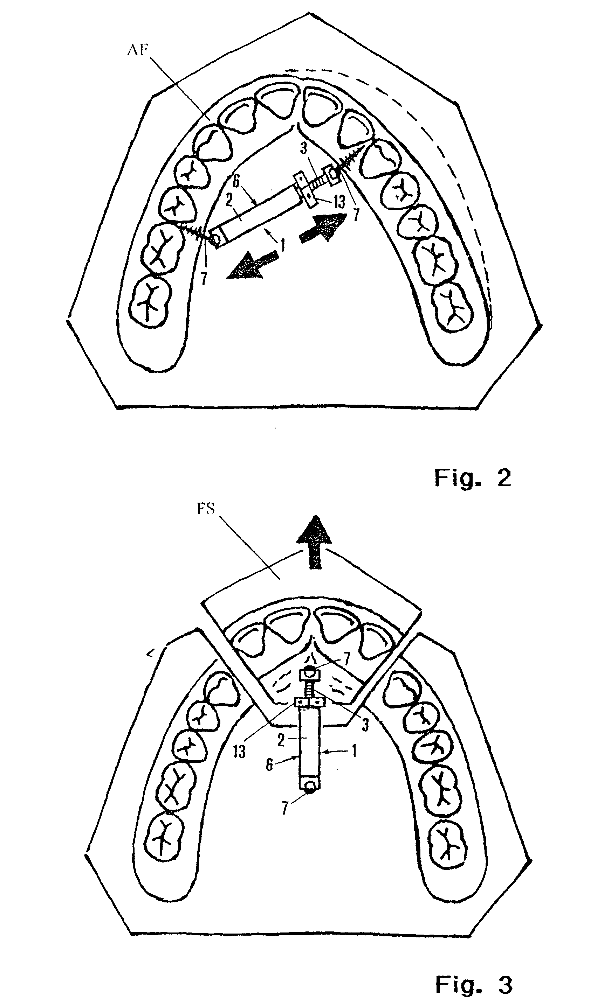

In Figur 2 ist anhand eines Gipsmodells die Anwendung des Distraktors 1, wie er auch in den Figuren 1a und 1b gezeigt ist, in schräger Anordnung im Gaumen gezeigt, um eine Asymmetrie des Alveolarfortsatzes AF des Oberkiefers, zum Beispiel in Folge einer Lippen-Kiefer-Gaumenspalte (auch Hasenscharte genannt) auszugleichen und den deformierten Teil des Alveolarfortsatzes gegen den Zug der Weichteile sofort in die richtige Position zu bringen. Die Distraktion wird so lange fortgeführt, bis die Knochenteile in der korrekten Okklusion ausgerichtet sind; danach können die Knochenteile in der erreichten Stellung mittels geeigneter Platten oder Schraubelemente, die nicht dargestellt sind, zusammengeschraubt werden.

Figur 3 zeigt den Einsatz eines einzelnen Distraktors, um ein Frontsegment, mit FS bezeichnet, des Oberkiefers graduell nach vorne zu verschieben, zum Beispiel um ein Missverhältnis zwischen Oberkiefer- und Unterkieferbezahnung auszugleichen oder um einen sogenannten frontalen Engstand aufzulösen, bei dem die Zähne mangels Platz verdreht oder verschachtelt stehen. Mit der graduellen Verschiebung des Frontzahnsegments entsteht ein zusätzlicher Knochen, in den die verdrehten Zähne mit kieferorthopädischen Methoden verschoben und damit der Engstand aufgehoben werden kann. Bei diesem Distraktor wird die eine Knochenschraube in dem Frontsegment FS verschraubt, während die andere Knochenschraube im Bereich der Sutur des harten Gaumens verankert ist.

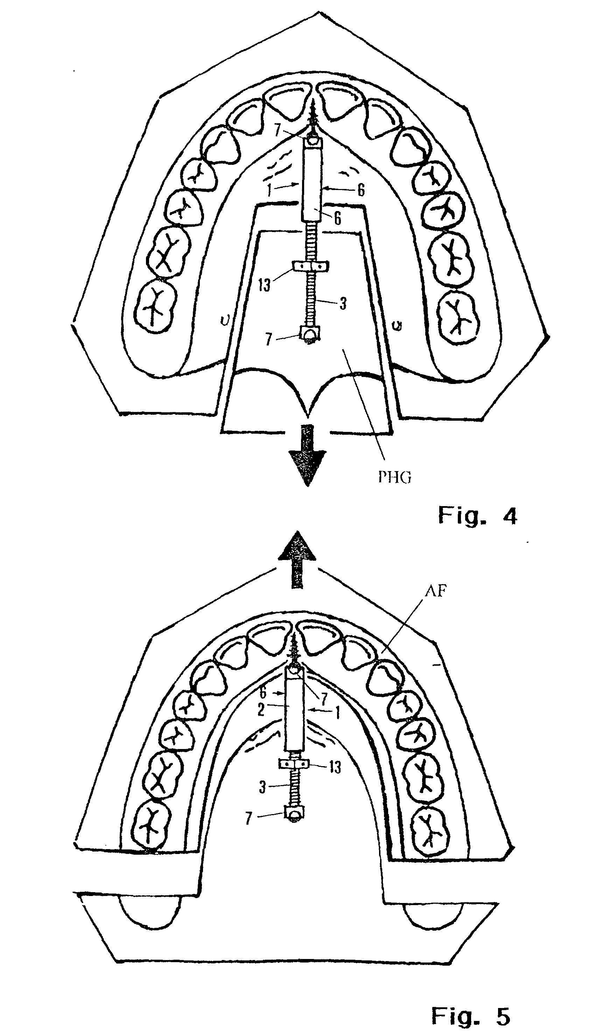

In der Anwendung, wie sie anhand des Gipsmodells in der Figur 4 gezeigt ist, wird der posteriore Teil des harten Gaumens, mit PHG bezeichnet, mit dem Distraktor 1 nach hinten verschoben, wie durch den Pfeil angedeutet ist. Dies kann dann notwendig sein, wenn die kongenitale velopharyngeale Inkompetenz besteht, was wiederum oft bei Lippen-Kiefer-Gaumenspalten der Fall ist. Der Patient ist bei Vorliegen dieses Problemes beim Sprechen behindert. Durch die graduelle Rückverlagerung des posterioren harten Gaumens kann das Problem in geeigneten Fällen behoben werden. Die Anordnung des Distraktors 1 ist hierbei entsprechend der Anordnung der Figur 3 gewählt.

In Figur 5 wird, im Vergleich zu der Anordnung der Figur 4, praktisch der ganze Alveolarfortsatz AF vom Rest des Oberkiefers gelöst und mittels des Distraktors 1 graduell nach vorne distrahiert, zum Beispiel um eine Fehlpositionierung zwischen Ober- und Unterkieferbezahnung auszugleichen.

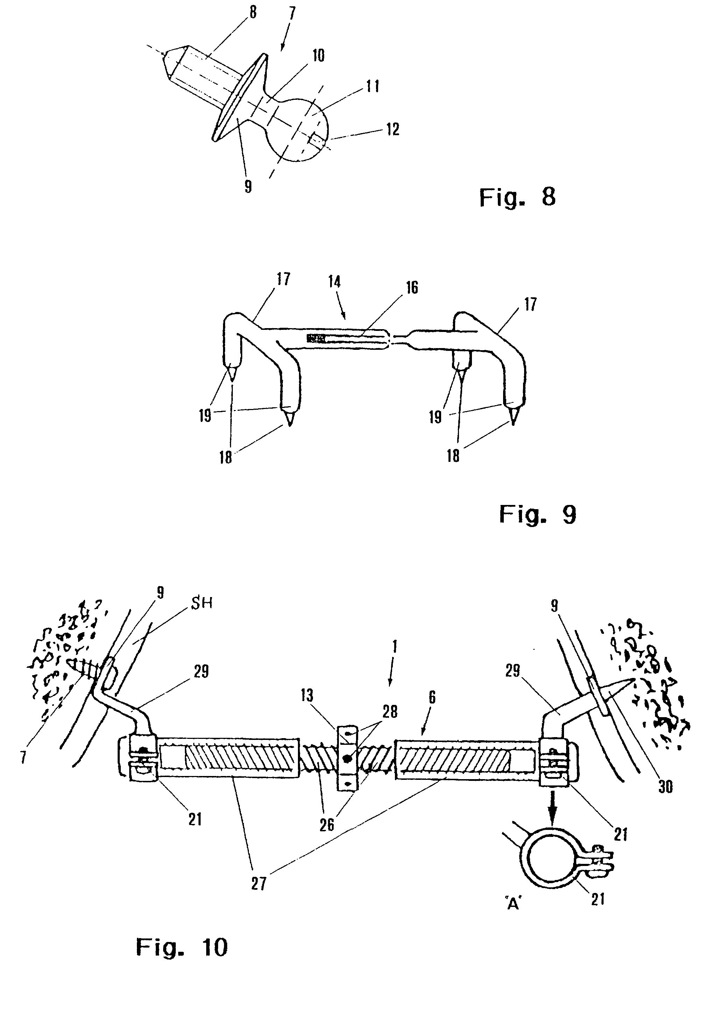

In der Darstellung der Figur 6a wird ein einzelner Distraktor 1 eingesetzt, um den Oberkiefer zu verbreitern. Dabei wird der Distraktor nicht auf Knochenschrauben 7 mit Kugelkopf abgestützt, sondern es werden zwei Bügel 14 eingesetzt; ein solcher Bügel ist auch in einer vergrösserten Darstellung in Figur 9 gezeigt. Die Verbindung zwischen Distraktor 1 bzw. Extensionsteil 6 und Bügel 14 erfolgt über Verbindungselemente 15 (siehe Figur 6a), die auf dem Bügelquerteil, mit 16 bezeichnet, an beliebiger Position festgeklemmt werden können und dieselbe Kugel wie die Gelenkkugel 11 der Knochenschraube 7 (siehe Figur 8) aufweisen, so dass das Extensionsteil 6 kompatibel eingesetzt werden kann.

Wie die Figur 9 zeigt, besitzt der Bügel 14 an den Enden des Bügelquerteils 16 zwei U-förmige Elemente 17, die an den freien Enden der beiden Schenkel jeweils einen Dornfortsatz 18 aufweisen. Der Dornfortsatz 18 ist an seiner Basis im Durchmesser geringer als die Stirnfläche 19 der Schenkel der U-förmigen Elemente 17, so dass eine Stufe entsteht, die ein Eindringen dieses Bügels in den Knochen begrenzt. Weiterhin ist in Figur 9 ersichtlich, dass das Bügelquerteil 16 geteilt ist mit einer Gewindeverbindung oder einer einfachen Steckverbindung (ohne Gewinde), so dass die beiden Hälften gegeneinander verdreht werden können, um das Bügelquerteil 16 zu verlängern oder zu verkürzen, um den jeweiligen Gegebenheiten anpassen zu können.

Die Extensionsteile 6, wie sie in den Figuren 1 bis 6 eingesetzt sind, sind in einer vergrösserten, detaillierten Darstellung in den Figuren 7a und 7b gezeigt. Wie bereits vorstehend erwähnt, handelt es sich bei dem Extensionsteil 6 des Distraktors 1 um eine Kombination von ineinandergreifenden Innen- und Aussengewindestangen bzw. Röhren oder Hülsen 2, 3. Gegenüber dem Extensionsteil 6, wie es vorstehend beschrieben ist, ist in der detaillierteren Darstellung der Figur 7a die Gewindestange 3 nochmals in einen Röhrenabschnitt 19 und einen Stangenabschnitt 20 unterteilt, so dass durch jeweiliges Einschrauben des Stangenabschnitts 20 in den Röhrenabschnitt 19 eine Grundeinstellung der Länge des Extensionsteils 6 erzielt werden kann. Folglich hat der Stangenabschnitt 20 ein Aussengewinde, das in dem Röhrenabschnitt 19 mit Innengewinde eingreift. Der Röhrenabschnitt 19 besitzt gleichzeitig ein Aussengewinde, das in das hülsenförmige Gewindeelement 2 mit Innengewinde eingreift. Die zwei Gewindepaare der Teile 20/19 und 19/2 sind so gegenläufig aufgebaut, dass durch Drehen des mittleren Teils 19 über die Sechskantmutter 13 das gesamte Extensionsteil 6 entweder verkürzt oder expandiert werden kann. Für die Verbindung des Extensionsteils 6 an den Knochenschrauben 7 mit Gelenkkugel 11 erfolgt über Klemmteile 21. Ein solches Klemmteil 21, wie es auch in Figur 7b gezeigt ist, die eine Ansicht aus Richtung des Sichtpfeils A in Figur 7a zeigt, ist ein kugelförmiger Hohlbereich 22, der auch als Gelenkpfanne bezeichnet werden kann, ausgebildet, der die Gelenkkugel 11 umfassen kann, so dass diese beim Eindringen quasi einschnappt. Die notwendige Elastizität, um die Gelenkkugel 11 zu klemmen, wird durch Anbringen eines Schlitzes 23 quer zu der Längsachse des Extensionsteils 6, die mit LA bezeichnet ist, erzielt. Die eigentliche Klemmung erfolgt dann über eine Schraubverbindung 24, die einen Schlitz 25 des Klemmteils 21 zusammenziehen kann, um die Gelenkkugel 11 festzuklemmen.

Figur 10 zeigt eine weitere Variante des Distraktors, wobei bei dieser Darstellung zwei

Möglichkeiten von Knochenabstützungsteilen zu sehen sind. In diesem Aufbau besitzt das Extensionsteil 6 eine Gewindestange mit zwei gegenläufigen Gewinden 26, zwischen denen die Sechskantmutter 13 zum Drehen der Gewindestange aufgesetzt ist. Auf jedes der gegenläufigen Gewinde 26 ist eine Hülse mit entsprechendem Innengewinde 27 aufgesetzt. Durch Drehung der Sechskantmutter 13 kann das Extensionsteil in die eine oder die andere Richtung verlängert oder verkürzt werden. Wie in Figur 10 zu sehen ist, weist die Sechskantmutter 13 sich radial erstreckende Bohrungen 28 auf, in die ein Handhabungswerkzeug zum Drehen der Mutter eingesteckt werden kann. Auf der Aussenseite der beiden Hülsen 27 ist wiederum ein Klemmteil 21 aufgesetzt, das in seinem Grundaufbau dem Klemmteil entspricht, wie es in Figur 7b gezeigt ist. Von diesem Klemmteil 21 erstreckt sich ein Arm 29, der abgewinkelt ist und an seinem Ende entweder eine Knochenplatte 9a für eine oder mehrere Standard-Knochenschraube(n) 7 (linke Seite) oder einen Dorn 30 (rechte Seite) mit Abstützungsscheiben 9 vorgesehen, um die Eindringtiefe der Knochenabstützungsteile zu begrenzen. In Figur 10 ist mit SH die Schleimhaut bezeichnet. Die Knochenschraube 7 wird, wie Figur 10 verdeutlicht, durch eine Stichinzision in der Schleimhaut SH in den Knochen eingetrieben. Die Arme 29 sind so abgewinkelt, dass die Knochenschraube 7 bzw. der Dorn 30 in etwa orthograt zu der entsprechenden Knochenoberfläche des harten Gaumens eingetrieben werden kann. Durch die Möglichkeit, dass der Arm 29 über das Klemmteil 21 an beliebiger Stelle und in einer beliebigen Winkelposition um die Längsachse des Extensionsteils 6 festgeklemmt werden kann, wird die Flexibilität und Adaptierbarkeit an die individuelle Knochentopographie stark verbessert.

Der Einsatz von Armen 29 mit Knochenplatten 9a ist speziell bei dünnen Knochen von Vorteil, bei dem die spezielle Schraube 2 gemäss Figur 8 nicht genügend fassen. Ausserdem ist durch die flexible Positionierung des Dorns oder der Knochenschrauben ausserhalb der direkten Achse des Distraktors mehr Platz für diesen vorhanden, bei den oft schmalen Gaumenwölbungen, da dieser praktisch unmittelbar bis an die Schleimhaut geführt werden kann.

Es ist darauf hinzuweisen, dass in den einzelnen Figuren Bauteile, die in den einzelnen Ausführungsformen in ihrer Funktionsweise vergleichbar sind, mit entsprechenden Bezugszeichen bezeichnet sind, so dass die Ausführungen zu den einzelnen Figuren analog auch auf die anderen Figuren übertragen werden können.

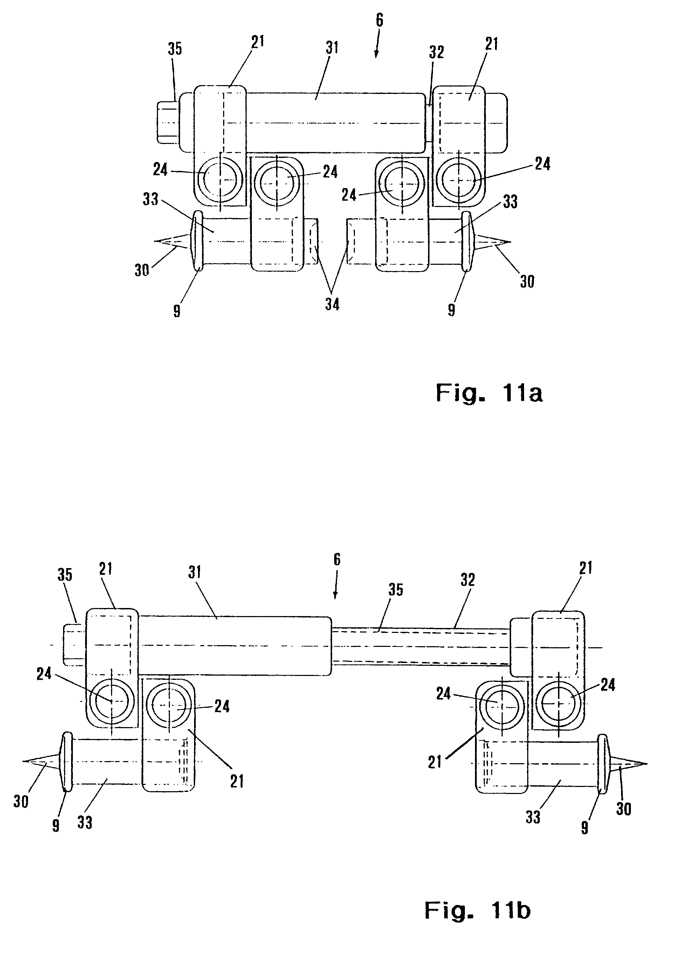

Die Figuren 11a und 11b zeigen eine weitere Ausführungsform eines Distraktors mit einem im Grundaufbau dreiteiligen Extensionsteil 6 mit einem zylindrischen Hülsenteil 31, mit einem Verlängerungsteil 32 und einem in dem Hülsenteil 31 geführten Gewindeelement 35, das an seinem linken, vorstehenden Ende eine Mehrkant-Aussenkontur aufweist, um dieses Gewindeelement über diesen Mehrkant zu drehen. Dieses Gewindeelement 35 ist in das Verlängerungsteil 32, das ein Innengewinde aufweist, hineingeschraubt, wie anhand der unterbrochenen Linien zu erkennen ist, so dass durch Drehen an dem Mehrkant das Verlängerungsteil 32 aus dem Hülsenteil 31 herausgeschoben, d.h. verlängert, oder hineingezogen werden kann, d.h. verkürzt werden kann. Das Hülsenteil 31 ist drehsicher gehalten. Gleiches gilt für das Verlängerungsteil 32. An den jeweiligen Enden der Hülse 31 und der Gewindestange 32 sind Klemmteile 21 befestigt, die mittels Schraubverbindungen 24 festgeklemmt werden können. Ein zweites Klemmteil 21 ist über eine geeignete Querverbindung mit dem ersten Klemmteil 21 verbunden und kann an der Querverbindung mittels Schraubverbindung 24 festgeklemmt werden. In diesem zweiten Klemmteil 21 ist ein Dorn 30, der ein zylindrisches Verlängerungsteil 32 besitzt, eingeklemmt. Das zylindrische Verlängerungsteil 33 kann in einer geeigneten Position an dem ihm zugeordneten Klemmteil 21 verklemmt werden, so dass das Extensionsteil 6 über dieses zylindrische Verlängerungsteil 33 zusätzlich in seinen Dimensionen geändert werden kann.

Die Figur 11a zeigt den Distraktor in einer zusammengeschobenen Stellung mit kleinster Dimension in Achsrichtung, während die Figur 11B den Distraktor in seiner maximal verlängerten Position zeigt. Wie in Figur 11a zu erkennen ist, sind die Stirnseiten der zylindrischen Verlängerungsteile 33, die dem Dorn 30 gegenüberliegen, mit einer konkaven Einwölbung 34 versehen. In die beiden Einwölbungen 34 der sich gegenüberliegenden zylindrischen Verlängerungsteile 33 kann ein Werkzeug, beispielsweise in der Art einer Zange, eingreifen, um die jeweiligen zylindrischen Verlängerungsteile 33 auseinanderzutreiben und die jeweiligen Dorne 30 in den Knochen an den geeigneten Stellen zu verankern, bevor das Extensionsteil durch Drehung der Gewindestange 32 verlängert wird.

In dieser Ausführungsform sind die Achsen des Extensionsteils (Teile 31, 32 und 35) zu den Achsen der zylindrischen Verlängerungsteile 33 versetzt, so dass sich ein Aufbau ergibt, bei dem das Extensionsteil 6 zu den Knochenverankerungspunkten versetzt ist.

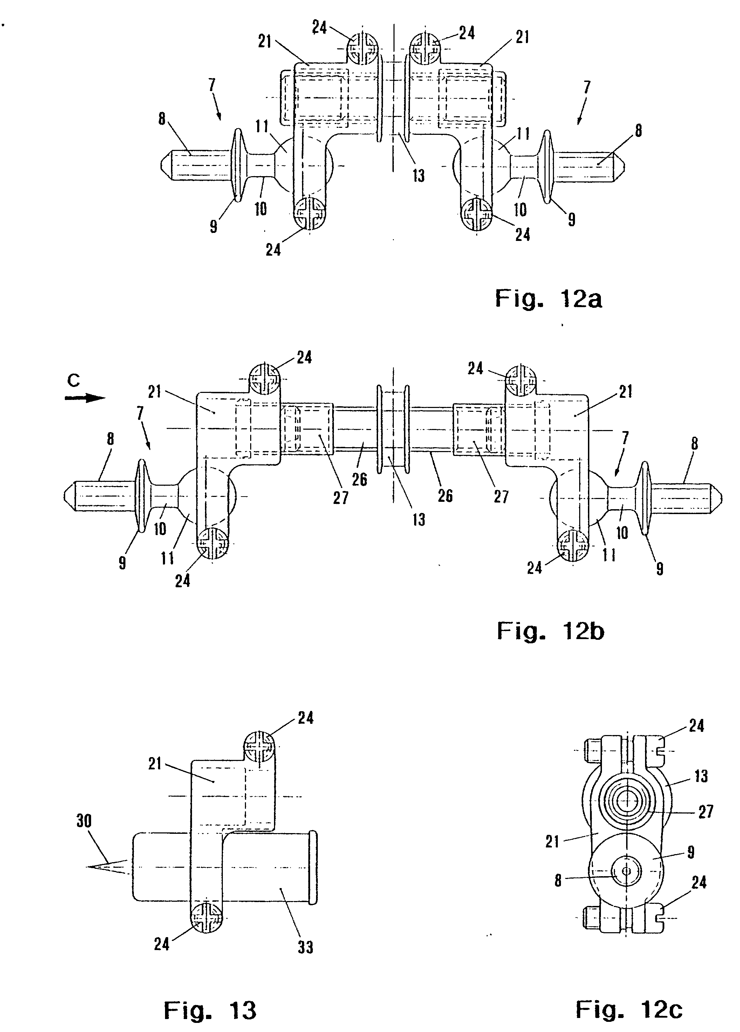

In den Figuren 12a bis 12c ist ein weiterer Distraktor gezeigt, der in seinem Grundaufbau demjenigen Distraktor entspricht, der in Figur 10 gezeigt ist. Die Klemmteile 21 sind, gegenüber der Ausführungsform der Figur 10, so aufgebaut, dass sie die Gelenkkugel 11 einer Knochenschraube 7, wie sie in Figur 8 gezeigt ist, aufnehmen können, und zwar entsprechend einem Aufbau, wie er in Figur 7a gezeigt ist. Allerdings sind in diesem Beispiel aufgrund der speziellen Klemmteile 21 die Achsen des Extensionsteils 6 und der Knochenschrauben 7 zueinander versetzt. Weiterhin können die Klemmteile 21 über die jeweiligen Klemmschrauben 36 an den Hülsen 27 verschoben und fixiert werden, wie anhand der unterschiedlichen Positionierungen der Klemmteile 21 in den Figuren 12a und 12b ersichtlich ist. Die jeweilige Hülse 27 und das jeweilige Klemmteil 21 sind über einen Formschluss so miteinander verbunden, dass das Klemmteil 21 nicht von der Hülse 27 abfallen kann.

In Figur 13 ist das Klemmteil 21 gezeigt, wie es in den Figuren 12a und 12b eingesetzt ist, allerdings mit einem Dorn 30 und einem zylindrischen Verlängerungsteil 33, entsprechend der Ausführungsform der Figur 11b, die anstelle der Knochenschraube 7 der Figuren 12a und 12b eingesetzt werden kann, falls der Distraktor nicht im Knochen verschraubt werden soll.

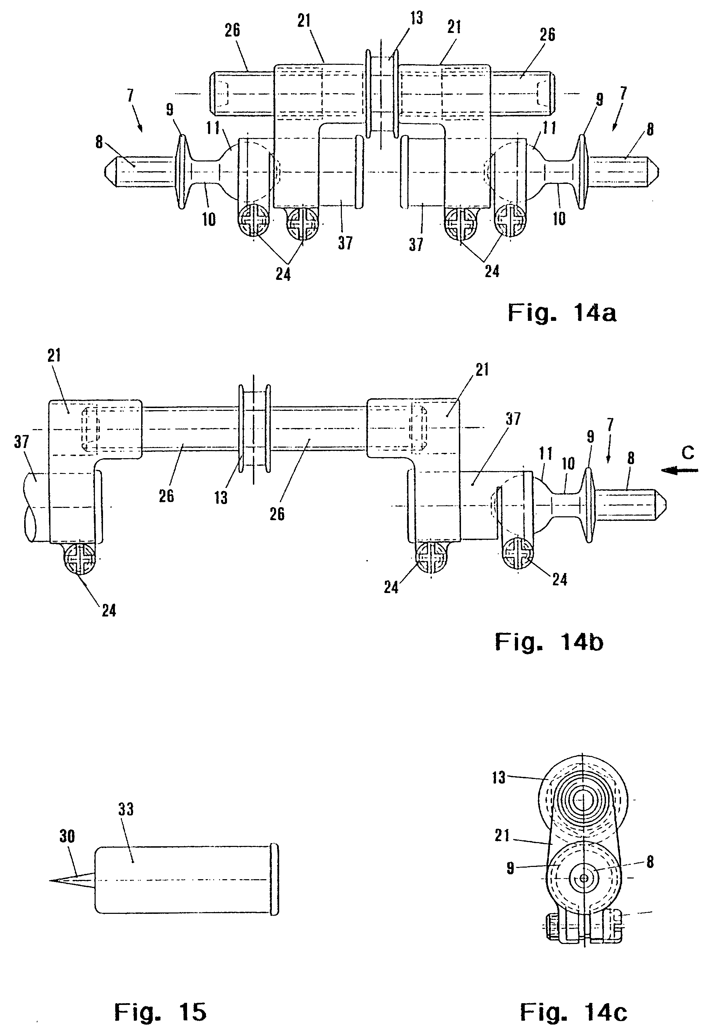

In der Ausführungsform der Figuren 14a bis 14c ist an den beiden Hälften der Gewindestange 26 beidseitig der Sechskantmutter 13, die jeweils wiederum gegenläufige Aussengewinde aufweisen, unmittelbar die Klemmteile 21, die hierzu entsprechende Innengewinde aufweisen, aufgesetzt. Ansonsten entsprechen diese Klemmteile denjenigen, die in Figur 13 gezeigt sind, wobei allerdings in den Klemmteilen 21 ein speziell ausgebildetes, zylindrisches Verlängerungselement 37 vorgesehen ist, das an seinem äusseren Ende mittels einer Klemmeinrichtung versehen ist, um darin die Gelenkkugel 11 einer Knochenschraube aufzunehmen. Der Aufbau dieses Klemmteils entspricht demjenigen, wie es in der Figur 7a an den beiden Enden des dort gezeigten Extensionsteil 6 angeordnet ist. Anstelle der zylindrischen Verlängerungselemente 37 kann ein zylindrisches Verlängerungsteil 33 mit Dorn 30, wie es in der Figur 15 gezeigt ist, eingesetzt werden, so dass sich mit diesem Aufbau eine grosse Variabilität des Distraktors ergibt.

Die Distraktoren, die in den Figuren 11, 12 und 14 gezeigt sind, haben als gemeinsames Merkmal die versetzten Achsen des Extensionsteils 6 zu den Achsen der Knochenabstützungselemente in Form der Knochenschrauben 7 oder der Dorne 30.

## Patentansprüche

1. Vorrichtung für die Gaumenerweiterung mit einem in seiner Längenabmessung verlängerbaren und/oder verkürzbaren Extensionsteil, das an seinen beiden Enden jeweils ein Fixierteil aufweist, **dadurch gekennzeichnet, dass** jedes Fixierteil (7, 18, 30) als punktförmiges Knochenabstützungsteil aufgebaut ist.

2. Vorrichtung nach Anspruch 1, **dadurch gekennzeichnet, dass** das Knochenabstützungsteil mit dem Extensionsteil (6) über ein einstellbares und in seiner Position fixierbares Gelenk (11) verbunden ist.

3. Vorrichtung nach Anspruch 1, **dadurch gekennzeichnet, dass** das Knochenabstützungsteil als Knochenschraube (7) ausgebildet ist.

4. Vorrichtung nach Anspruch 1, **dadurch gekennzeichnet, dass** das Knochenabstützungsteil als Dorn (18, 30) ausgebildet ist.

5. Vorrichtung nach Anspruch 1, **dadurch gekennzeichnet, dass** das Knochenabstützungsteil (7, 18, 13) eine die Eindringtiefe in den Knochen begrenzende Verdickung (9) aufweist.

6. Vorrichtung nach Anspruch 5, **dadurch gekennzeichnet, dass** die Verdickung (9) tellerartig ausgebildet ist.

7. Vorrichtung nach Anspruch 5, **dadurch gekennzeichnet, dass** die Verdickung wulstartig ausgebildet ist.

8. Vorrichtung nach Anspruch 1, **dadurch gekennzeichnet, dass** das Extensionsteil (6) aus mindestens drei Gewindeelementen (2, 3, 4; 2, 3, 19; 26, 27, 27; 26, 21,21) aufgebaut ist, die miteinander verschraubt zwei gegenläufige Gewindepaare bilden, wobei eine Drehung des mittleren Gewindeelements (3; 19; 26) in der einen bzw. der anderen Drehrichtung das Extensionsteil (6) verlängert oder verkürzt.

9. Vorrichtung nach Anspruch 1, **dadurch gekennzeichnet, dass** das Extensionsteil (6) aus mindestens drei Elementen (31, 32, 35) aufgebaut ist, die miteinander verbunden sind, wobei das erste Element (31) hülsenförmig ausgebildet und darin drehbar gelagert das zweite Element (35) aufnimmt, und wobei das dritte Element (32) mit dem zweiten Element (35) so verschraubt ist, dass das dritte Element (32) unter Drehung des zweiten Elements (35) in die eine bzw. in die andere Richtung das dritte Element (32) in das erste Element (31) hineingezogen oder aus diesem herausgeschoben wird und damit das Extensionsteil (6) verkürzt oder verlängert wird.

10. Vorrichtung nach Anspruch 1, **dadurch gekennzeichnet, dass** die Achsen der Knochenabstützungsteile (7, 30) zu der Achse des Extensionsteil (6) versetzt sind.

11. Vorrichtung nach Anspruch 10, **dadurch gekennzeichnet, dass** die Knochenabstützungsteile (7, 30) mittels jeweils einer Klemmteil (21) an dem Extensionsteil (6) gehalten ist.

12. Vorrichtung nach Anspruch 10, **dadurch gekennzeichnet, dass** das jeweilige Klemmteil (21) an dem Extensionsteil lösbar und verschiebbar ist.

13. Vorrichtung nach Anspruch 11, **dadurch gekennzeichnet, dass** das Knochenabstützungsteil (7, 30) an dem Klemmteil (21) lösbar und verschiebbar gehalten ist.

14. Vorrichtung nach Anspruch 10, **dadurch gekennzeichnet, dass** das Knochenabstützungsteil (30) an seinem Ende, das dem Knochenanlageende gegenüberliegt, eine Druckfläche (34) bildet.

15. Vorrichtung nach Anspruch 14, **dadurch gekennzeichnet, dass** die Druckfläche als Vertiefung (34) ausgebildet ist.

16. Vorrichtung nach Anspruch 2, **dadurch gekennzeichnet, dass** das Gelenk durch ein Kugelgelenk (11, 22) gebildet ist, wobei die Kugel (11) des Gelenks dem Knochenabstützungsteil (7) zugeordnet ist.

17. Vorrichtung nach Anspruch 2 und Anspruch 16, **dadurch gekennzeichnet, dass** die Kugel (11) in der Pfanne (22) durch eine Klemmschraube (24) festklemmbar ist oder, durch Lösen der Klemmschraube (24), drehbar gelagert verbleibt.

18. Vorrichtung nach Anspruch 8 und Anspruch 11, **dadurch gekennzeichnet, dass** zwei der Gewindeelemente durch Teile der beiden Klemmteile (21) gebildet sind.

19. Vorrichtung nach Anspruch 18, **dadurch gekennzeichnet, dass** das jeweilige Gewindeelement des Klemmteils (21) als eine Hülse mit Innengewinde ausgebildet ist.

## Claims

1. Apparatus for the expansion of the palate with an extension part which may be lengthened and/or shortened in its length, which has on each of its two ends a fixation part, **characterized in that** each fixation part (7, 18, 30) is constructed in the form of a single-point bone support element.

2. Apparatus according to claim 1, **characterized in that** said bone support element is connected to the extension part (6) by an adjustable and in its position fixable joint (11).

3. Apparatus according to claim 1, **characterized in that** said bone support element takes the form of a bone screw (7).

4. Apparatus according to claim 1, **characterized in that** said bone support element takes the form of a pin (18, 30).

5. Apparatus according to claim 1, **characterized in that** said bone support element (7, 18, 13) comprises a shoulder (9) limiting the impression depth into bone.

6. Apparatus according to claim 5, **characterized in that** said shoulder (9) takes the form of a disk.

7. Apparatus according to claim 5, **characterized in that** said shoulder is in the form of a rim.

8. Apparatus according to claim 1, **characterized in that** said extension part (6) consists of at least three threaded elements (2, 3, 4; 2, 3, 19; 26, 27, 27; 26, 21, 21) which form when screwed into each other two counter running pairs of threads where turning the middle thread element (3, 19, 26) in the one or the other direction of rotation is lengthening or shortening the extension part (6).

9. Apparatus according to claim 1, **characterized in that** the extension part (6) is built from at least three elements (31, 32, 35) which are connected to each other, wherein the first element (31) takes the form of a tube which receives interior the second element remaining turnable and wherein the third element (32) is screwed to the second element in such a way that the third element (32) by turning the second element (35) in one or the other direction is pulled into the first element (31) or pushed out of it and therefore the extension part (6) is lengthened or shortened.

10. Apparatus according to claim 1, **characterized in that** the axes of said bone support elements (7, 30) are offset with respect to the axis of the extension part (6).

11. Apparatus according to claim 10, **characterized in that** the bone support elements (7, 30) are each fixed with a clamping part (21) on the extension part (6).

12. Appratus according to claim 10, **characterized in that** the corresponding clamping part (21) is detachable and movable on said extension part.

13. Apparatus according to claim 11, **characterized in that** said bone support elements (7, 30) is fixated on the clamping part (21) in a detachable and movable connection.

14. Apparatus according to claim 10, **characterized in that** the bone support element (30) forms a surface for pressure application (34) on its end opposite to the bone contact area.

15. Apparatus according to claim 14, **characterized in that** the surface for pressure applications takes the form of a groove (34).

16. Apparatus according to claim 2, **characterized in that** the joint is formed by a ball joint (11, 22), wherein the ball (11) of the joint is part of the bone support element part (7).

17. Apparatus according to claim 2 and claim 16, **characterized in that** the ball (11) may be clamped within the cup (22) by a clamping screw (24) or may remain rotatable by loosening the clamping screw (24).

18. Apparatus according to claim 8 and claim 11, **characterized in that** two of the threaded elements are formed by parts of the two clamping elements (21).

19. Apparatus according to claim 18, **characterized in that** the corresponding threaded element of the clamping part (21) takes the form of a tube with interior threads.

## Revendications

1. Dispositif pour l'extension de palais avec une partie d'extension pouvant être allongée et/ou raccourcie qui présente respectivement à ses deux extrémités une pièce de fixation, **caractérisé en ce que** chaque pièce de fixation (7, 18, 30) est construite comme pièce de soutien osseux ponctuel.

2. Dispositif selon la revendication 1, **caractérisé en ce que** la pièce de soutien osseux est reliée à la pièce d'extension (6) par une articulation réglable et fixable en position (11).

3. Dispositif selon la revendication 1, **caractérisé en ce que** la pièce de soutien osseux est réalisée comme une vis pour ostéosynthèse (7).

4. Dispositif selon la revendication 1, **caractérisé en ce que** la pièce de soutien osseux est réalisée comme une cheville (18, 30)

5. Dispositif selon la revendication 5, **caractérisé en ce que** la pièce de soutien osseux (7, 18, 13) présente une grosseur (9) limitant la profondeur de pénétration dans l'os.

6. Dispositif selon la revendication 5, **caractérisé en ce que** la grosseur (9) a la forme d'un plateau.

7. Dispositif selon la revendication 5, **caractérisé en ce que** la grosseur a la forme d'un bourrelet.

8. Dispositif selon la revendication 1, **caractérisé en ce que** la pièce d'extension (6) se compose d'au moins trois éléments filetés (2, 3, 4, ; 2, 3, 19; 26, 27, 27; 26, 21, 21) qui vissés l'un à l'autre forment deux paires filetées opposées, une rotation de l'élément fileté médian (3; 19; 26) allongeant ou diminuant dans un sens ou dans l'autre sens de rotation la pièce d'extension (6).

9. Dispositif selon la revendication 1, **caractérisé en ce que** la pièce d'extension (6) se compose d'au moins trois éléments (31, 32, 35) qui sont reliés entre eux, le premier élément (31) ayant la forme d'une gaine pouvant loger le second élément (35) en rotation et le troisième élément (32) étant vissé avec le second élément (35) de manière que le troisième élément (32) est introduit en tournant le seconde élément (35) dans un sens ou dans l'autre , dans le premier élément (31) ou en est retiré ce qui permet de rallonger ou de raccourcir la pièce d'extension (6).

10. Dispositif selon la revendication 1, **caractérisé en ce que** les axes des pièces de soutien osseux (7, 30) sont décalés par rapport à l'axe de la pièce d'extension (6).

11. Dispositif selon la revendication 10, **caractérisé en ce que** les pièces de soutien osseux (7, 30) sont maintenues au moyen d'une pièce de serrage (21) à la pièce d'extension (6).

12. Dispositif selon la revendication 10, **caractérisé en ce que** la pièce de serrage respective (21) est mobile et amovible sur la pièce d'extension.

13. Dispositif selon la revendication 11, **caractérisé en ce que** la pièce de soutien osseux (7, 30) est mobile et amovible sur la pièce de serrage (21).

14. Dispositif selon la revendication 10, **caractérisé en ce que** la pièce de soutien osseux (30) forme une surface de pression (34) á son extrémité qui est opposée à l'extrémité d'application osseuse.

15. Dispositif selon la revendication 14, **caractérisé en ce que** la surface de pression est réalisée comme une cavité (34).

16. Dispositif selon la revendication 2, **caractérisé en ce que** l'articulation est formée par une rotule (11, 22), la rotule (11) de l'articulation étant associés á la pièce de soutien osseux (7).

17. Dispositif selon la revendication 2 ou la revendication 16, **caractérisé en ce que** la rotule (11) est fixable dans le cotyle (22) par une vis de serrage (24) ou reste logée en rotation par desserrage de la vis de serrage (24).

18. Dispositif selon la revendication 8 ou la revendication 11, **caractérisé en ce que** deux des éléments filetés sont formés par des pièces des deux éléments de serrage (21).

19. Dispositif selon la revendication 18, **caractérisé en ce que** l'élément de serrage respectif de la pièce de serrage (21) est réalisé comme un manchon avec un filet
